# EUROPEAN PATENT SPECIFICATION

(11) **EP 1 418 922 B1**
(45) Date of publication and mention of the grant of the patent: **18.06.2008**
(21) Application number: 02754804.9
(22) Date of filing: 01.07.2002
(51) Int. Cl.: A61K 31/683, A61K 31/685, A61P 27/02, A61K 9/08, A61K 9/10, A61K 9/127

(54) **USE OF NEGATIVELY CHARCHED PHOSPHOLIPIDS FOR THE PREPARATION OF A MEDICAMENT FOR TREATMENT AND/OR PREVENTION OF MACULAR DEGENERATION**
VERWENDUNG VON NEGATIV-GELADENEN PHOSPHOLIPIDEN ZUR HERSTELLUNG EINES MEDIKAMENTS ZUR BEHANDLUNG UND/ODER VORBEUGUNG VON MAKULADEGENERATION
UTILISATION DES PHOSPHOLIPIDES CHARGES NEGATIVEMENT POUR LA MANUFACTURE D'UN MEDICAMENT POUR TRAITER ET/OU A PREVENIR LA DEGENERESCENCE MACULAIRE

(30) Priority: 22.08.2001 DE 10141018
(43) Date of publication of application: 19.05.2004
(73) Proprietor: LYNKEUS BIOTECH GmbH, 97076 Würzburg (DE)
(72) Inventor: RICHTER, Christoph, CH-8044 Zürich (CH); GAZZOTTI, Paolo, CH-8954 Geroldswil (CH); SHABAN, Hamdy, CH-5605 Dottikon (CH)
(74) Representative: Vossius & Partner
(86) International application number: PCT/EP2002/007237
(87) International publication number: WO 2003/018028

(56) References cited:
- EP-A- 0 312 814
- EP-A- 0 459 148
- EP-A- 0 531 933
- WO-A-00/35438
- WO-A-90/11781
- WO-A-96/40092
- SUTER, M. ET AL.: "Age-related macular Degeneration" THE JOURNAL OF BIOLOGICAL CHEMISTRY, vol. 275, no. 50, 15 December 2000 (2000-12-15), pages 39625-39630, XP002216156 usa cited in the application
- SHABAN, H., RICHTER,C.: "A2E and Blue Light in the Retina: The Paradigm of Age-Related macular Degeneration" BIOLOGICAL CHEMISTRY, vol. 383, no. 3-4, March 2002 (2002-03) - April 2002 (2002-04), pages 537-545, XP001106922
- SHABAN, H. ET AL.: "Cytochrome c Oxidase Inhibition by N-Retinyl-N-retinylidene Ethanolamine, a compound Suspected to Cause Age-Related Macula Degeneration" ARCHIVES OF BIOCHEMISTRY AND BIOPHYSICS, vol. 394, no. 1, 1 October 2001 (2001-10-01), pages 111-116, XP001105978
- HRUBY, K., WIESFLECKER, J.: "Trockene senile Makulopathie. Prophylaxe und Therapie in Risikofällen" KLINISCHE MONATSBLÄTTER FÜR AUGENHEILKUNDE, vol. 182, no. 6, June 1983 (1983-06), pages 570-575, XP001106747
- WEISS, H., KOSMATH, B.: "Therapeutische Anwendung von Phosphatiden bei retinalen Erkrankungen" KLINISCHE MONATSBLÄTTER FÜR AUGENHEILKUNDE, vol. 158, no. 2, February 1971 (1971-02), pages 278-285, XP001106740

## Description

### BACKGROUND OF THE INVENTION

The present invention relates to negatively charged phospholipids, as well as uses including negatively charged phospholipids and possibly carotenoids and/or antioxidants, for treating the eye. In a preferred embodiment, the present invention relates to the use of negatively charged phospholipids for treating age-related macular degeneration.

### PROBLEMS OBSERVED IN PRIOR ART

Age-related macular degeneration (AMD) affects 10 to 20% of the population over 65 years old and represents one of the main causes of serious vision damage and/or vision problems of older people in the industrial nations (Klein, R., Klein, B.E., and Linton, K.L. (1992) Ophthalmology 99, 933-943). One differentiates between the wet form of macular degeneration, which affects approximately 20% of the patients and is treatable using photodynamic therapy, and the dry form of AMD, which affects approximately 80% of the patients. The dry form of AMD typically has a slow course and has not been treatable until now.

The molecular causes of this illness, which is particularly significant in geriatrics, have not been well researched. The newest investigations have shown that a pigment (A2E, N-retinyl-N-retinylidene ethanolamine), which forms naturally in the eye during the seeing process and increases tenfold with progressing age (Eldrid, G.E., Lasky, M.R. (1993) Nature 361, 724-726; Parish, C.A., Hashimoto, M., Nakanishi, K., Dylon, J., Sparrow, J. (1998) Proc. Natl. Acad. Sci. USA 95, 14609-14613), causes the death of pigment epithelial cells of the retina (Suter, M., Remé, C.E., Grimm, C., Wenzel, A., Jäättela, M., Esser, P., Kociok, N., Leist, M. and Richter, C. (2000) J. Biol. Chem. 275, 39625-39630). There are some indications that A2E is partially responsible for the dry form of AMD.

New investigations show that the cell toxicity of A2E is caused by an extremely specific mode of action (see Suter et al., ibid.): A2E obstructs the interaction of cytochrome c with cytochrome c oxidase in the mitochondria. This has dramatic consequences for the cell, since an interruption of the mitochondrial respiratory chain occurs and therefore a reduction of the energy turnover and the release of reactive oxygen. Furthermore, a release of cytochrome c into the cytoplasm occurs, so called apoptosomes being formed and apoptosis occurring, i.e., the programmed cell death sets in.

In the final analysis, however, the precise mechanisms which lead to programmed cell death in the macula, and therefore to the destruction of the macula and the reduction and/or complete loss of the ability to see in older patients, are still unexplained.

### OBJECT AND SUMMARY OF THE INVENTION

As a consequence, there is a strong desire in the related art to provide substances and compositions which may be used as medications in order to treat pathological conditions of the eye, and particularly AMD, so that prevention, alleviation, or healing of this illness is possible. There is particularly an increasing desire for these types of uses, since the average age of the population is increasing due to medical progress and therefore -- in relation to the total population -- more and more patients are affected by this illness. Furthermore, there is a desire to be able to treat pathological conditions of the retina caused by other processes, which have possibly not yet been explained. These primarily concern oxidative damage of the eye and/or the retina, as well as damage due to the effects of aggressive agents and/or high-energy radiation.

Accordingly, one object of the present invention is to provide uses which allow eye illnesses, particularly macular degeneration and AMD, to be treated and/or which allow prevention of these illnesses. Furthermore, it is an object of the present invention to provide substances and/or compositions and pharmaceutical preparations which may be used for treating macular degeneration in general and particularly for AMD.

Described herein is a composition for the treatment and/or prevention of macular degeneration and/or pathological conditions of the retina that are based on macular degeneration, comprising at least one negatively charged phospholipid except cardiolipin. The present invention relates to a use of such a composition for the manufacture of a medicament for the treatment and/or prevention of macular degeneration and/or pathological conditions of the retina that are based on macular degeneration. Also described is a method for the manufacture of such a medicament, wherein the composition, comprising at least one negatively charged phospholipid except cardiolipin is used as an essential constituent. A further method for producing such a composition includes the following steps: a) drying of a composition which is provided in a solvent, and b) reconstitution of the composition in a physiologically acceptable solution. Also described herein are eye drops, an eye bath, an eye insert, or a semisolid preparation for the treatment and/or prevention of macular degeneration and/or pathological conditions of the retina that are based on macular degeneration, wherein a composition comprising at least one negatively charged phospholipid except cardiolipin is used as an essential constituent. The invention relates to a use of such a composition for the treatment and/or prevention of macular degeneration and/or pathological conditions ,of the eye that are based on macular degeneration. Furthermore, the invention relates to the use of such a composition which is administered in a physiologically acceptable solution to the eye. Advantageous embodiments and additional characteristics in accordance with the invention ensue from the dependent claims.

The present invention relates to the use of negatively charged phospholipids for the preparation of a medicament or composition for treating and preventing macular degeneration and/or pathological conditions of the retina. In the framework of the present invention, "macular degeneration" is to be understood to mean a progressive destruction of the macula. This destruction and/or degeneration may be age-related (AMD), however, pathological processes which are not age-related are also conceivable.

Furthermore, target indications of the uses, substances, and compositions, and/or methods for their production, as described herein are pathologies of the eye and the retina, which may be triggered, for example, by damaging agents or radiation and which make pharmaceutical treatment necessary. These types of agents include, for example, aggressive acids, bases, radicals, and radical producers, as well as further irritative or pathogenic chemicals. These pathological conditions may be expressed in such a way that a progressive impairment of the seeing ability of younger patients occurs. Described also expressly concern this patient group. Furthermore, the described use also concerns the treatment of pathological processes on or inside the retina whose genesis is of an unexplained nature. Correspondingly, the phospholipids of the present invention (as well as mixtures of the same and the compositions according to the present invention, see below) may be used for treating various pathological conditions of the retina of differing genesis.

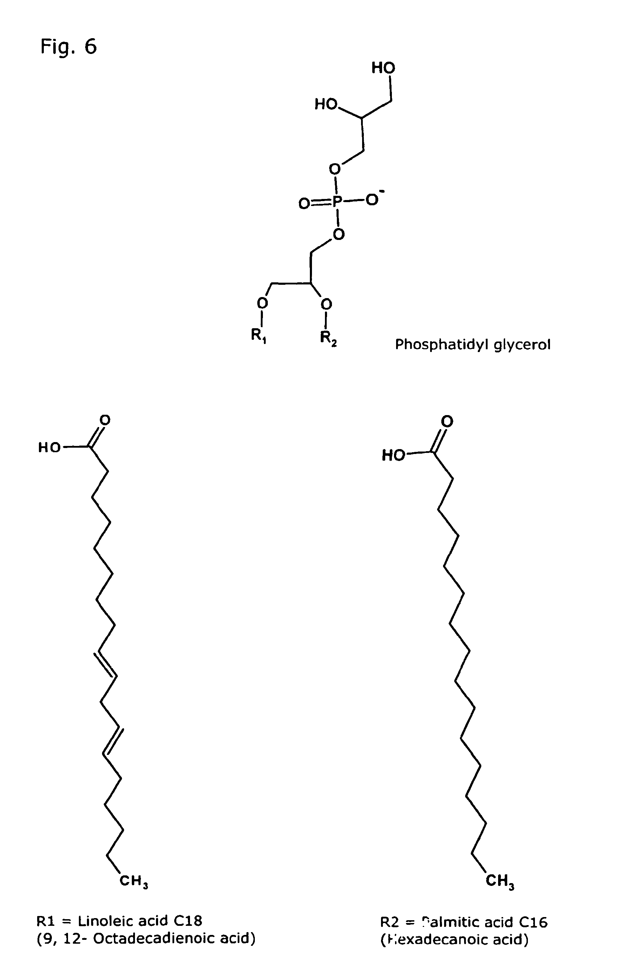

The present invention relates to a use in which the negatively charged phospholipid is selected from the group including phosphatidylinositol (PI), cardiolipin (CL, synonym: 1,3 diphosphatidylglycerol, DPG) and phosphatidylglycerol (PG). In a particularly preferred embodiment, phosphatidylglycerol is used to treat the eye and macular degeneration. Phosphatidylglycerol (PG) is a negatively charged phospholipid. Chemically it can be classified as an ester, i.e., a compound that is formed when alcohol(s) and acid(s) condense with elimination of water. In PG, the alcoholic parts are contributed by 2 glycerol moieties, the acidic parts by 1 phosphoric acid residue and by 2 fatty acid residues (designated R1 and R2 in the scheme, see Fig. 6). In naturally occurring PG, R1 and R2 are variable with respect to their length (normally a chain of 16 - 18 carbons) and their degree of saturation (saturated or unsaturated). It is possible to synthesize novel and unique PGs in which R1 and R2 are shorter or longer than those in naturally occurring PGs, and may be saturated or unsaturated. Such synthetic PGs promise to be useful drugs (see below).

The singular form, "a negatively charged phospholipid", refers to a species of molecules which each have the same head group. "Head group" is to be understood to mean the organic residue bonded to the phosphate group, the "anchor" (in the sense of a membrane anchor) of the phospholipid, in contrast, being formed by the fatty acids. The phospholipids having the same head group, which form a species, may, however, be individually esterified using fatty acids of different chain lengths, which may have a varying degree of saturation. This is preferred in the framework of the present invention, since naturally occurring biological systems (for example naturally occurring membranes) are also constructed in this way (see also explanations below).

The phospholipid systems described above are significantly more similar to the bodily lipid systems (e.g. of the retina) of the patient than, for example, synthetic phospholipids, which have a narrower melting range, since only one fatty acid or a few different fatty acids are esterified in them. Therefore, the compositions preferred according to the present invention are better suitable for being introduced into the biological system of the retina and exhibiting their healing effect there.

The use of the negatively charged phospholipid for treating the eye and macular degeneration includes preparations of the respective phospholipid which are pharmaceutically acceptable. The latter primarily indicates that the phospholipid is free from degradation products -- particularly those arising from oxidation-(e.g. free short-chain carboxylic acids and aldehydes), and/or the concentrations of these decomposition products are as low as possible. The guidelines of *"good manufacturing practice"* (GMP guidelines), which contain the provisions for adequate and safe production of pharmaceuticals and to which reference is explicitly made in the framework of the present invention, are helpful as a handbook in regard to the use according to the present invention. Furthermore, in addition to the GMP guidelines, the corresponding EU standards and provisions should be observed during the selection and provision of the phospholipids to be used, and during the use, provision, and production of the compositions and mixtures described in more detail below, so that production and use which are as pharmaceutically safe as possible and correspond to these standards is ensured.

Furthermore, it is to be noted in regard to the chain lengths that the individual negatively charged phospholipids occurring in a population may each be esterified on a molecular level using different fatty acids, so that only a statistical value may be indicated in regard to the overall chain length of a species of negatively charged phospholipids. Chain lengths which occur in natural products are preferred for the fatty acids bonded to the glycerol via an ester bond, natural products being understood, for example, to include lipids from (mammal) retina, plant sources, or fish. The advantages of this type of "mixed" construction of the fatty acids are the more favorable packing densities of the lipid molecules in the membrane and/or the vesicle (see below) obtained in this way, and in the transition range (and/or melting range) established in this way. In regard to the degree of saturation of the fatty acids occurring in the phospholipid, a higher degree of saturation (i.e., there are fewer C-C double bonds) of the esterified fatty acid molecules leads to a lower fluidity of the membrane and a higher melting point. For this reason, the preferred phospholipid mixtures to be used in the framework of the present invention are preferably esterified with a proportion of singly or multiply unsaturated fatty acids which corresponds to the proportion occurring in natural membranes of mammals, particularly preferably the proportion of the mammal retina. These types of (micro heterogeneous) compositions are typical for naturally occurring phospholipid compositions and are preferred in the framework of the present invention, since they have advantageous biological properties (i.e., biocompatibility) and a melting point and/or melting range -- the phase transition between crystalline and liquid lipid phase is meant here -- suitable for biological systems (which include the retina). This effect frequently does not occur in synthetic lipids and/or lipid mixtures, so that they may possibly not be universally usable in the framework of the present invention. In any case, their suitability in regard to phase transition temperature and biocompatibility should be investigated before use.

The present invention also relates to the use of a composition including at least two different negatively charged phospholipids as defined in the claims for treating macular degeneration and/or pathological conditions of the retina. The mixture ratio of the two negatively charged phospholipid components may be selected as desired in this case. In a preferred embodiment, mixture ratios are selected which have been shown to be particularly effective for the illness of the eye and/or the retina to be treated and/or which allow optimum mixing of the two negatively charged phospholipids. The principles already described above in regard to the use of one single phospholipid (i.e., one species) apply in regard to the chain lengths to be used and the degree of saturation of the fatty acids present in the phospholipid.

The present invention relates to a use in which the negatively charged phospholipids are selected from the group consisting of phosphatidylglycerol, phosphatidylinositol, and cardiolipin. The use of phosphatidylglycerol has been shown to be particularly suitable and therefore particularly preferred (see also exemplary embodiments).

Described herein is also the use of a composition, which includes a negatively charged phospholipid, at least one carotenoid, and possibly at least one antioxidant, to treat the eye or to prevent pathological conditions of the eye. The present invention relates to the use of a composition, which includes a negatively charged phospholipid as defined in the claims, at least one carotenoid, and possibly at least one antioxidant, to treat or to prevent macular degeneration and/or pathological conditions of the retina. The use of a phospholipid provided in a mixture with one or more carotenoids has been shown to be advantageous in the framework of the present invention. In a preferred embodiment of the present invention, lutein and zeaxanthin are used. The carotenoids may each be used individually, however, a mixture of lutein and zeaxanthin is particularly preferred. One possible reason for the advantageous use of one or more carotenoids is the protection obtained in this way of the lipids existing in the retina from oxidation and/or oxidative degradation by reactive oxygen species and other radicals, as well as a protective effect from the pathogenic effect of A2E. The latter may be explained using the filter effect of the carotenoids (they represent a blue light filter), which leads to shielding of the retina and the compositions used, which include phospholipids, from high-energy radiation. Naturally, the carotenoid(s) and possibly the antioxidant protect the phospholipid used even before introduction into the eye and/or into the retina. This elevates the storage stability of the compositions used. In the framework of the use according to the present invention, the carotenoid, the phospholipid, and possibly the antioxidant may be in any possible mixture ratio.

Furthermore, the present invention relates to the use of a composition, which includes a negatively charged phospholipid, at least one carotenoid, and possibly at least one antioxidant, in which the negatively charged phospholipid is selected from the group consisting of phosphatidylinositol, and phosphatidylglycerol. In the framework of the present invention, the use of phosphatidylglycerol (PG) in combination with at least one carotenoid has been shown to be particularly advantageous. In a preferred embodiment of the present invention, lutein and/or zeaxanthin are used as carotenoids.

In a further advantageous embodiment, the present invention relates to the use of a composition, which includes at least two different negatively charged phospholipids and at least one carotenoid and possibly at least one antioxidant, to treat the eye. The present invention also relates to the use of a composition, which includes at least two different negatively charged phospholipids, at least one carotenoid, and possibly at least one antioxidant, to treat or to prevent macular degeneration and/or pathological conditions of the retina. An embodiment of the uses according to the present invention described above is preferred in which the antioxidant and/or the oxidants are selected from the group including ubiquinone (coenzyme Q10), vitamin E, and ascorbic acid, ubiquinone being particularly preferred.

The present invention further relates to uses in which the composition includes, in addition to the negatively charged phospholipids, neutral and/or positively charged phospholipids. Asolectin, a phospholipid mixture from soybeans, which includes a high proportion of negatively charged phospholipids (approximately 15%), as well as further neutral and/or positively charged phospholipids, represents a mixture preferred in the framework of the present invention. In the framework of the present invention, all phospholipids whose net charge is "0" at neutral pH (i.e., pH 7.0 at 25 °C and biochemical standard conditions) are referred to as neutral phospholipids, i.e., their head groups either exist completely without charges (such as in phosphatidylserine, PS), as a zwitterion, or in such a way that positive and negative charges compensate one another. Corresponding phospholipids whose net charge is positive under the conditions described above (such as in phosphatidylcholine, PC) are referred to as positively charged phospholipids. The same definitions as those which were already used above in connection with the negatively charged phospholipids apply here in regard to the chain lengths and the degree of saturation. In general, it is known to those skilled in the art that the use of further neutral and/or positively charged phospholipids may be advantageous or even necessary, in order that the negatively charged phospholipids of the present invention are optically effective and well-tolerated in the eye and/or in the retina. Thus, for example, the addition of further phospholipids (neutral or positively charged) is frequently necessary in order to allow a use as a liposome and/or lipid vesicle, since exclusively negatively charged head groups repel one another and therefore the membrane structure, among other things, is destabilized, so that further lipids must be used as a "matrix". Such a (phospho)lipid which is frequently used in membrane biochemistry is, for example, phosphatidylcholine (PC) from chicken eggs ("egg PC"). It is clear to one skilled in the art without anything further that the use of a mixture of negatively charged phospholipids according to the present invention and/or antioxidants with PC represents a nearly *in vivo* system, whose use may be advantageous in relation to the use of exclusively negatively charged phospholipids. In an advantageous embodiment, the additional neutral and/or positively charged phospholipids are mixed with negatively charged phospholipid (and/or phospholipids) before use. An advantageous mixture ratio (neutral and/or positively charged phospholipids : negatively charged phospholipids) is approximately 2:1, approximately 1:1 is preferred, and a mixture ratio of 1:2 to 1:5 is particularly preferred. Furthermore, the present invention relates to uses which additionally include lutein and/or zeaxanthin. The present invention also relates to compositions which include at least one negatively charged phospholipid and at least one neutral and/or positively charged phospholipid.

Also described herein are compositions in which the negatively charged phospholipid or the negatively charged phospholipids are selected from the group including phosphatidylinositol, cardiolipin, phosphatidylglycerol, and asolectin, as well as compositions including a mixture of at least one negatively charged phospholipid, at least one neutral and/or positively charged phospholipid, at least one carotenoid, and possibly at least one antioxidant. Furthermore, described is the use of the compositions described above for treating the eye, particularly macular degeneration and/or pathological conditions of the retina.

Finally, described herein is a method for producing one of the compositions described above, which includes the following steps:
a) drying of a composition described above, which is provided in a suitable solvent, and
b) reconstitution of the composition in a physiologically acceptable solution;
c) possibly further treatment of the solution according to step b), so that lipid vesicles and/or liposomes are formed.

The method for producing the compositions described here is a method which essentially includes the steps of drying and reconstitution. The concept "drying" (step a) is to be understood here in such a way that the solvent containing the composition is removed. This preferably occurs under sterile conditions and using methods known in the related art, such as drawing off the solvent under vacuum and subsequent drying under high vacuum. In a preferred embodiment of the present invention, the drawing off of the solvent is performed In such a way that a lipid film which is as thin as possible remains in the vessel containing the composition (deposition of the phospholipid). A lipid film which is as thin as possible has the advantage that residual solvent possibly still present may be removed without problems (by evaporation), while the deposition of thicker films has the disadvantage that residual solvent still present is more difficult to remove in the time interval given. The mixing of different phospholipids, possibly in combination with carotenoid(s) and/or antioxidants, preferably occurs while these compounds are still in the solvent, since mixing after storage and reconstitution in buffer (see below) causes a less homogeneous mixing of the solution. Organic solvents are suitable as a solvent, those solvents which have a low toxicity in trace amounts being particularly preferred (e.g. absolute ethanol, clinical purity grade). Of course, care must be taken in the production method according to the present invention that work is done in accordance with the GMP guidelines and/or the European Pharmaceutical Guidelines, so that a pyrogen-free composition without toxic impurity components, which has a high degree of physiological compatibility, is obtained.

In step b) of the method, reconstitution of the composition in a physiologically acceptable solution is achieved. "Reconstitution" is to be understood to mean dissolving the compositions including phospholipid in buffer. "Dissolving" or "solution" are also to be understood in the framework of the present invention to mean the suspension of the lipids in a solution. In a preferred embodiment, this is performed by adding glass microspheres to the vessel containing the lipid film, rotating the glass microspheres on the lipid film in the presence of the physiologically acceptable solution, and subsequent freezing and thawing ("freeze thaw") of the suspension (e.g., freezing in liquid nitrogen, thawing in the water bath). In this way, large multilayered vesicles form which include the composition according to the present invention and possibly enclose reconstitution solution. These vesicles may be freed from larger lipid aggregates by brief centrifuging (10-30 seconds, 4000 RPM, Heraeus Laboratory Centrifuge). In addition, filtration and/or sterile filtration may be performed. In order to obtain an even more defined vesicle population, extrusion of the lipid solution through a membrane having a defined pore diameter may also be performed (step c). The solution may be extruded 10 to 20 times through a sterile membrane, care being taken that the finished phospholipid (vesicle) solution is removed from the side lying opposite the inlet side of the membrane, since in this way lipid aggregates and "giant vesicles" are held back on the side of the membrane facing away from the finished solution. Suitable devices for extruding lipid solutions and/or suspensions are known to those skilled in the art (mini extruder, French press, etc.). In addition to the generation and use of liposomes and/or vesicles, step b) also includes the reconstitution of the lipids as a "crude" suspension, i.e., the reconstitution is performed without techniques being used which are directed toward generating liposomes or vesicles. Furthermore, the present invention also includes the use of mild detergents in order to reconstitute the lipids in the physiologically acceptable solution. The further treatment in the framework of step c) alternatively also includes other methods known to those skilled in the art for producing liposomes and/or vesicles, such as cholate dialysis methods, as well as further methods for purification, homogenization, and clarification of the composition including the lipids.

Furthermore, described is the use of a composition obtainable according to one of the methods described above for treating the eye, particularly macular degeneration and/or pathological conditions of the retina. Also described herein is the use of a composition obtainable according to one of the methods described above as eye drops, eye baths, eye inserts, or semisolid preparations for application onto the eye. In this connection, care is to be taken that the use as eye drops, eye baths, eye inserts, or semisolid preparations for application onto the eye makes necessary the measures already described above in regard to the production of pharmaceuticals in accordance with the guidelines. Reference is made in this connection to the remarks already made above in this connection, and to the general principles listed in the following in regard to the production of eye drops, eye baths, eye inserts, or semisolid preparations for application onto the eye.

Also disclosed are eye drops, eye baths, eye inserts, or semisolid preparations for application onto the eye which include a negatively charged phospholipid, as well as eye drops, eye baths, eye inserts, or semisolid preparations for application onto the eye which include at least two different negatively charged phospholipids. Eye drops in the sense of the present invention are aqueous or oily solutions for application in or on the eye. Sterile and/or bacteria-free and pathogen-free solutions or suspensions including the negatively charged phospholipid(s) of the present invention, and further ingredients (carotenoid(s), antioxidant(s), see below), are preferably used.

Eye baths In the sense of the present invention are aqueous solutions for bathing and washing the eye or for soaking eye bandages. In a preferred embodiment, sterile and/or bacteria-free and pathogen-free solutions or suspensions including the negatively charged phospholipid(s) of the present invention, and further ingredients (carotenoid(s), antioxidant(s), see below), are used. Eye inserts in the sense of the present invention are represented by solid or semisolid preparations of suitable size and shape which are introduced into the conjunctival sac in order to produce an effect on and/or in the eye. In a preferred embodiment, this occurs through a successive release of the agents and ingredients of the composition, including the negatively charged phospholipids, into the conjunctival sac, so that the composition may diffuse and/or flow onto the location to be treated. In the framework of the present invention, salves, creams, or gels are semisolid preparations.

Furthermore, described herein are eye drops, eye baths, eye inserts, or semisolid preparations for application onto the eye which include a negatively charged phospholipid and at least one carotenoid, and possibly at least one antioxidant, as well as eye drops including at least two different negatively charged phospholipids, at least one carotenoid, and possibly at least one antioxidant. The present invention also relates to eye drops, eye baths, eye inserts, or semisolid preparations for application onto the eye which include at least one of the compositions described above. The eye drops, eye baths, eye inserts, or semisolid preparations for application onto the eye according to the present invention may include supplemental ingredients which, for example, improve the tonicity or viscosity of the preparation, adjust or stabilize the pH value (buffer substances), elevate the solubility of the phospholipid, antioxidant, or carotenoid, or preserve the preparation.

Furthermore, also described is a method for treating the eye, characterized by the application of a physiologically acceptable solution, which includes a negatively charged phospholipid, into the eye of the patient. In the framework of the present invention, the use of PG in combination with a carotenoid is preferred. Sterile, sterile filtrated, and pyrogen-free physiological saline solution is preferred as a physiologically acceptable solution. The concept of "solution" is to be understood here in such a way that suspensions of lipids or lipid vesicles and/or aggregates in the physiologically acceptable solution are also included. Furthermore, semisolid preparations, such as creams, salves, or gels, are also to be understood as "solutions" in the sense of the present invention. However, alternative solutions, which are physiologically acceptable and are suitable in the framework of the use and production of eye drops, eye baths, eye inserts, or semisolid preparations for application onto the eye, are also known to those skilled in the art, particularly to physicians. The concept of "application" is to be understood here in such a way that methods known to those skilled in the art for introducing a solution into the eye are applied. These include the administration of eye drops, eye baths, eye inserts, or semisolid preparations for application onto the eye and/or solutions, washes, and injections on suitable locations.

Furthermore disclosed is a method for treating or preventing macular degeneration and/or pathological conditions of the eye, particularly the retina. This method is distinguished by the administration of a negatively charged phospholipid to the body in physiologically acceptable ways that are known to those skilled in the art, e.g. via intravascular applications or extravascular applications including oral, intranasal and transdermal administration.

Also described is a method for treating or preventing macular degeneration and/or pathological conditions of the retina, distinguished by the application of a physiologically acceptable solution, which includes a negatively charged phospholipid, into the eye of the patient. The method may be used to treat pathological conditions of the retina and macular degeneration, particularly AMD.

Also described is a method in which the negatively charged phospholipid is selected from the group including phosphatidylglycerol, phosphatidylinositol, and cardiolipin, a method in which the physiologically acceptable solution includes at least two different negatively charged phospholipids, and a method in which the physiologically acceptable solution additionally includes at least one carotenoid and possibly at least one antioxidant. Finally, a method is disclosed in which the physiologically acceptable solution includes additional neutral and/or positively charged phospholipids. The present invention will be described with reference to the diagrams, tables, and examples. The diagrams and examples are not to be understood as limiting, but are used for a more detailed explanation of the uses according to the present invention.

### BRIEF DESCRIPTION OF THE FIGURES

- Fig. 1: shows the concentration dependence of the inhibition of solubilized cytochrome c oxidase by A2E;
- Fig. 2: shows the dependence on illumination of the inhibition of solubilized cytochrome c oxidase by A2E:
Fig. 2a shows the time course of inactivation;
Fig. 2b shows the effect of preincubation in the light followed by activity measurements in the dark;
Fig. 2c shows oxygen supplementation;
Fig. 2d shows recovery by 1,3 DPG;
- Fig. 3: shows the effect of the particularly preferred phosphatidylglycerol on the activity of cytochrome c oxidase, as described in exemplary embodiment 2;
- Fig. 4: shows the dependence of the inhibition of reconstituted cytochrome c oxidase by A2E on the phospholipid used;
- Fig. 5: shows the dependence on illumination of the inhibition of reconstituted cytochrome c oxidase by A2E;
- Fig. 6: shows naturally occurring phosphatidylglycerol (PG), the acidic parts being contributed by 1 phosphoric acid residue and by 2 fatty acid residues (R1 and R2, normally having a chain of 16 - 18 carbons).

### DETAILED DESCRIPTION OF THE INVENTION

### 1. Materials and methods used

### A) Materials:

A2E was synthesized from all*-trans* retinal and ethanolamine (Parish, C. A., Hashimoto, M., Nakanishi, K., Dillon, J., and Sparrow, J. (1998) Proc. Natl. Acad. Sci. USA 95, 14609-14613) and purified as described using chromatography on silica gel (Suter, M., Remé, C.E., Grimm, C., Wenzel, A., Jäättela, M., Esser, P., Kociok, N., Leist, M., and Richter, C. (2000) J. Biol. Chem. 275, 39625-39630). [³H]-A2E (specific activity 4.4 Ci/mol) was synthesized using [1-³H] ethane-1-ol-2-amine hydrochloride (Amersham Schweiz). Peroxynitrite was produced as described (Kissner, R., Beckman, J.S., and Koppenol, W. (1996) Methods Enzymol. 269, 296-302) and was made available by Dr. R. Kissner, Institute for Inorganic Chemistry, ETH Zürich. The remaining chemicals used had the highest possible degree of purity and were obtained by purchase from appropriate firms.

Cytochrome c oxidase was purified from rat liver mitochondria of female Wistar rats as described by Frei *et al.* (Frei, B., Winterhalter, K.H., and Richter, C. (1985) J. Biol. Chem. 260, 7394-7401). The homogenization medium contained 210 mM mannitol, 70 mM saccharose, 10 mM tris-HCl (pH 7.4), 0.1 mM EDTA, and 0.5 mg/mL BSA. The heart mitochondria were purified using a similar method, a polytron homogenizer being used. The cytochrome c oxidase was purified according to the method described by Adez and Cascarano (Adez, I.Z., and Cascarano, J. (1977) J. Bioenerg. Biomemb. 9, 237-253). Cytochrome c oxidase solubilized using Triton X-100 was reconstituted in variously composed phospholipid vesicles of differing dimensions, a modified cholate dialysis method being used. (Niggli, V., Siegel, E., and Carafoli, E. (1982) J. Biol. Chem. 257, 2350-2356). 1 mL of a phospholipid suspension (10 mg/mL in 50 mM KCI, 10 mM HEPES-KOH (pH 7.0), 0.1 mM K⁺-EDTA; buffer A) was solubilized in 0.2 mL of a 400 mM cholate solution. The purified cytochrome c oxidase (40 µg) was added to 0.4 mL of the cholate-phospholipid mixture. After an hour on ice, the sample was put into dialysis tubes and dialyzed in the cold against 1 L of buffer A (24 hours), three buffer replacements being performed. Cytochrome c oxidase from *Paracoccus denitrificans* was graciously provided by Dr. Bernd Ludwig, Biology Center of the University of Frankfurt am Main, Germany.

### B) Methods:

The cytochrome c oxidase activity was determined by establishing the oxygen consumption. For this purpose, a Clarke electrode was used at 25°C (Yellow Spring Instruments, Yellow Spring, OH), the measurement being performed under continuous stirring. Solubilized cytochrome c oxidase was suspended in 40 mM K⁺-phosphate, 0.005 mM EDTA (pH 7.0), 0.1% Tween 20 containing 0.02 mM cytochrome c as a substrate and a mixture of ascorbate und tetramethyl-p-phenylenediamine (TMPD) (10 mM/1 mM). Reconstituted cytochrome c oxidase was measured with ascorbate/TPMD (10 mM/0.2 mM) in a medium containing 100 mM KCl, 0.01 M HEPES-KOH (pH 7.0) und 0.1 mM EDTA. The maximum activity (V max) was determined by destroying the membrane proton gradient using 2 µM carbonyl cyanide-3-chlorophenyl hydrazone und 0.8 µg valinomycin/(mL of test medium).

The radiation with light was performed using a 70 watt lamp which was attached at 10 cm height over the electrode. The oxidation of lipids using peroxynitrite was achieved by suspending 20 mg DPG in 1 mL of 40 mM K⁺-phosphate and 0.05 mM EDTA (pH 7.0) and mixing with 0.94 µmol peroxynitrite (original solution 94 mM in 0.1 N NaOH). Malondialdehyde was measured as an index for the lipid oxidation as already described (Klein, S.D., Walt, H., and Richter, C. (1997) Arch. Biochem. Biophys., 348, 313-319).

The binding of A2E on cytochrome c oxidase was measured as follows: [³H]-A2E (final concentration 40 µM) was added to solubilized or reconstituted cytochrome c oxidase or to other proteins. The quantity of protein used in each case was approximately 50 pmol. After 20 minutes of incubation in the darkness or under radiation using light, 10% (final concentration) of trichloroacetic acid was added, the precipitate was collected on Millipore filters, and the dissolved filter was examined for radioactivity.

### 2. Results

### Example 1: Inhibition of solubilized cytochrome c oxidase by A2E

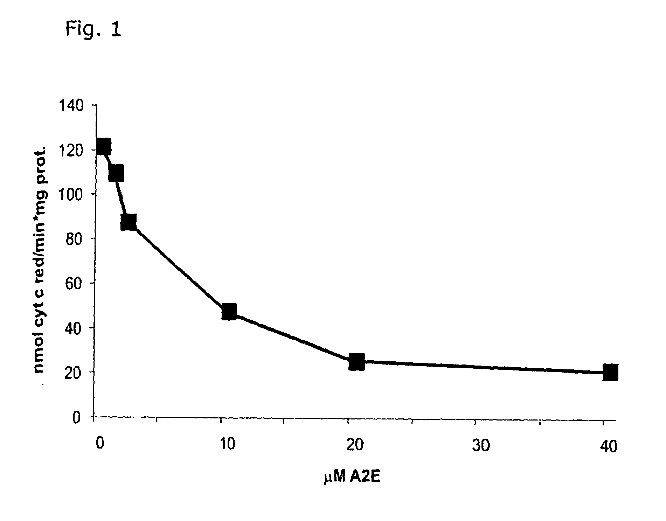

The inhibition of solubilized cytochrome c oxidase (COX) by A2E was measured. The activity of the COX could be easily measured by registering the oxygen consumption of the enzyme in the presence of ascorbate and TPMD (tetramethyl-p-phenylene diamine) as an electron source to reduce cytochrome c. It was shown that the extent of COX inhibition depended both on the quantity of enzyme and on the A2E concentration used. The A2E concentration necessary to achieve 50% inhibition (i.e., the IC₅₀ value) was 3 µM for 1 µg COX/mL, 7 µM for 2 µg COX/mL (see Fig. 1) and 20 µM for 4 µg COX/mL. The activity of solubilized cytochrome c oxidase (c = 2 µg/mL) was measured in a Clarke electrode in the presence and absence of A2E.

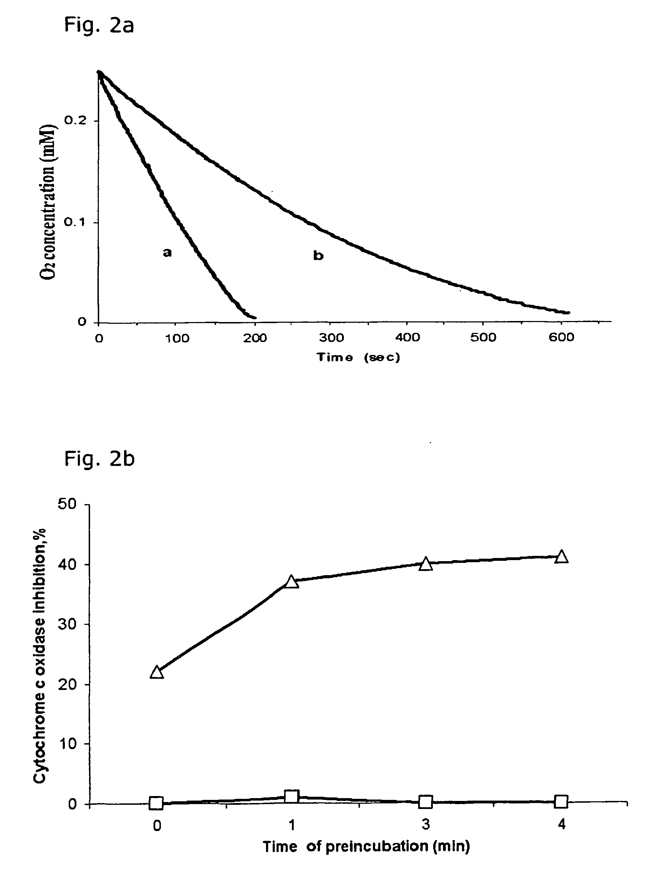

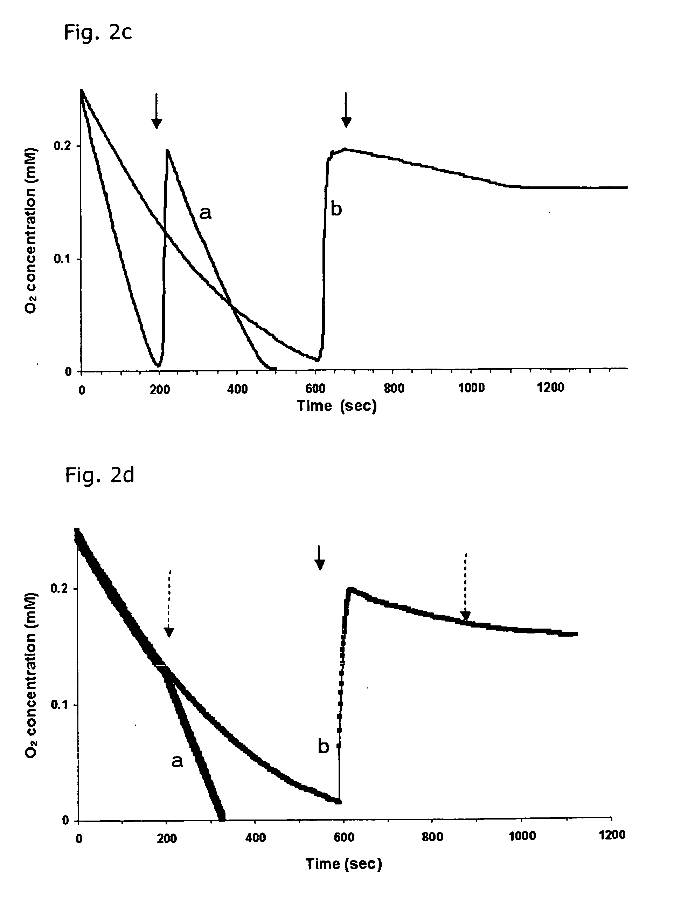

Furthermore, the time dependence of the inactivation of COX by A2E was measured (Fig. 2). The oxygen consumption of solubilized cytochrome c oxidase (c = 4 µg/mL) was measured in a Clarke electrode in the presence of A2E in darkness and/or while being exposed to light, as described in Example 2. Fig. 2a) shows the time dependence of the inactivation in darkness (line a) and/or under the incidence of light (line b). Fig. 2b) shows the effect of pre-incubation under the incidence of light with 20 µM A2E (triangles) or without A2E (squares), followed by activity measurements in darkness. Fig. 2c) shows the oxygen supplementation. The experiment was performed in darkness (line a) and/or under the incidence of light (line b). 5 µg of catalase from beef liver with 500 µM of H₂O₂ was added at the positions marked with arrows. Fig. 2d) shows the recovery phase with cardiolipin under the incidence of light. Two separate measurements are illustrated (lines a and b). At the positions marked by dashed arrows, 300 µg/mL of cardiolipin was added at t = 210 seconds and t = 950 seconds. At the position of the solid arrow, 5 µg of catalase from beef liver with 500 µM of H₂O₂ was added. It should be noted that in the absence of A2E, cytochrome c oxidase is equally active in darkness and under the incidence of light (data not shown).

In darkness, COX is already partially inactivated by A2E. Under the incidence of light, a progressive inactivation of the COX may be measured (Figs. 2a and 2b). Control experiments showed that this was not connected to damage of cytochrome c, ascorbate, or TPMD by light and A2E. The inactivation of the COX was also not based on a change of its affinity to oxygen, since the supplementation of O₂ after its complete consumption did not stimulate the enzyme (Fig. 2c). Rather, the complete inactivation of COX was finished by A2E through further exposure to light (Fig. 2c). The activity of the COX may be partially restored if cardiolipin is added in an earlier stage (Fig. 2d), however, this is not true if cardiolipin is added later.

### Example 2: Specificity of the COX inhibition by A2E, as well as dependence of the inhibition of solubilized cytochrome c oxidase on the phospholipid used and prevention of the inhibition by negatively charged phospholipids

It was further investigated which substances prevented or weakened the inactivation induced on COX by A2E. For this purpose, first the effect of multiple cationic and lipophilic substances on the IC₅₀ value of COX was investigated (see Table I).

**Table I:**

| **Lipophilic/cationic compound** | **IC₅₀ (µM) in relation to COX** |
|---|---|
| Stearylamine | No inhibition |
| Dequalinium | 40 |
| TPP⁺ | No inhibition |
| Retinal | 200 |
| Tamoxifen | 100 |
| MPP⁺ | No inhibition |
| A2E | 7 |

Table I shows the activity of solubilized COX in the presence of cationic and/or lipophilic substances. The oxygen consumption of solubilized COX (2 µg/mL) was measured in the presence of various substances using a Clarke electrode (see materials and methods). Whenever possible, an IC₅₀ value was determined, i.e., the concentration which is necessary to achieve 50% inhibition. TPP⁺ = tetraphenyl phosphonium ion; MPP⁺ = methylphenyl pyridinium ion.

It was shown that the IC₅₀ value of COX was quite high when most of these compounds were used, or that, among other things, there was even no inhibition of the COX. This shows that the substances, particularly dequalinium and tamoxifen, do cause inhibition of the COX, but do not act nearly as specifically as A2E, which has the lowest IC₅₀ value by far (7 µM) and should therefore be considered a highly specific inhibitor of COX.

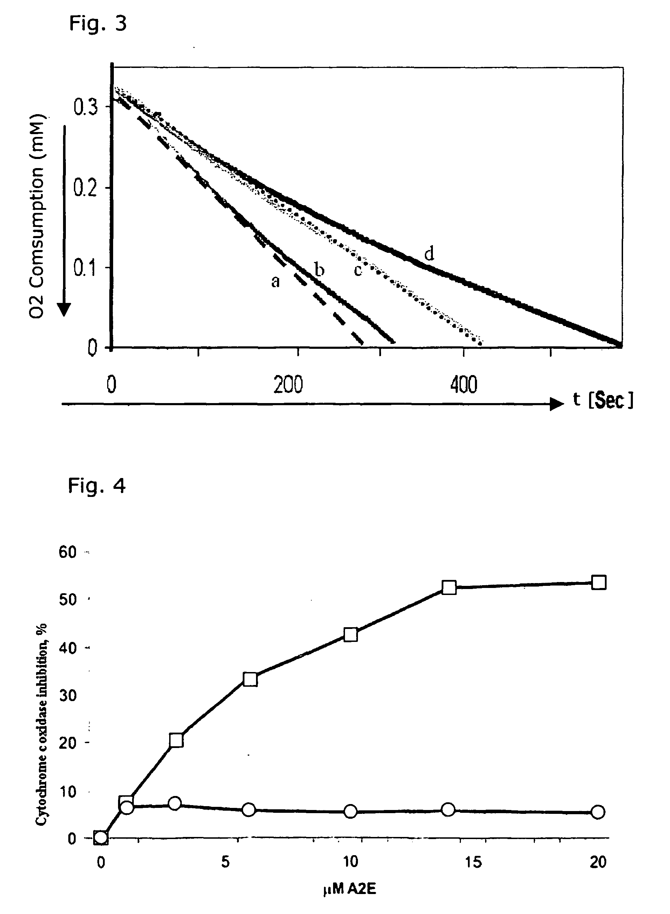

Furthermore, it was found that the negatively charged phospholipid cardiolipin (CL) reduced the inhibition of COX by A2E (see also Fig. 3, second-highest curve c). In this case, the oxygen consumption of COX in the presence of PG (300 µg/mL) and A2E (40 µM) [curve b], CL (300 µg/mL) and A2E (40 µM) [curve c], A2E only (40 µM) [curve d], as well as without additional substances (control: only COX) [curve a] are graphed against time. The polycations ruthenium red and La³⁺, which are known to interact strongly with CL, were used as a control.

In fact, it was shown that when these substances were added the protective effect of CL was no longer present (see Table II).

**Table II**

| **Addition of** | **% COX inhibition** |
|---|---|
| 20 µM A2E | 60 |
| A2E + CL (200 µg/mL) | 0 |
| A2E + CL (200 µg/mL) + RR (10 µM) | 60 |
| A2E + CL (200 µg/mL) + La³⁺ (200 µM) | 60 |

Table II shows the activity of solubilized COX in the presence of A2E, cardiolipin, and polycations. The oxygen consumption of solubilized COX (2 µg/mL) was measured in the presence of various substances using a Clarke electrode (see materials and methods). The A2E concentration was always 20 µM. CL = cardiolipin (diphosphatidylglycerol, DPC); RR = ruthenium red.

The protective effect of CL could also be removed if it was (pre)treated using peroxynitrite, an effective oxidant, which is formed in mitochondria. It is probable that during a treatment of this type the double bonds of the fatty acids are first oxidatively attacked and subsequently short-chain carboxylic acids are formed as decomposition products. A treatment of this type leads to the loss of the protective properties of CL, and promotion of the COX inhibition even occurs. The effect of a mixture of negatively charged phospholipids on the A2E-induced inhibition of COX was investigated. For this experiment, asolectin was used, a mixture of phospholipids from soybeans, which contains approximately 15% acid (i.e., negatively charged at pH 7.0) phospholipids. The use of asolectin also led to pronounced protection of COX in the presence of A2E (Table III).

**Table III**

| **Phospholipids** | **Decoupled COX inhibition [%]** |
|---|---|
| Asolectin | 0 |
| PC:PE(1:1,w/w) | 70 |
| PC:PE:CL (1:1:0.01) | 45 |
| PC:PE:CL (1:1:0.1) | 32 |
| PC:PE:CL (1:1:0.25) | 9 |
| PC:PE:CL (1:1:0.5) | 0 |

Table III shows the activity of COX reconstituted in vesicles made of various phospholipids. Solubilized COX was reconstituted in various types of phospholipid vesicles. The activity of the enzyme was measured in the presence of 14 µM of A2E, as described (see above), in a Clarke electrode. PC = phosphatidylcholine; PE = phosphatidylethanolamine; CL = cardiolipin (diphosphatidylglycerol, DPC).

A further phospholipid which is particularly preferred in the framework of the present invention is phosphatidylglycerol (PG). The effect of phosphatidylglycerol in the presence of A2E on the COX activity was measured. It may be seen from Fig. 3 that in the presence of A2E alone, i.e., without protective phospholipid, the COX activity is the lowest, which may be seen from the lower O₂ consumption per unit of time (curve d). In the presence of cardiolipin, whose use is advantageous in the framework of the present invention (see above), a protective effect is shown on the COX (curve c), since in this case the oxygen consumption of the COX per unit of time is already elevated in the presence of A2E and CL and therefore a higher COX activity exists than without negatively charged phospholipid (cf. curve d). The control (COX only) shows elevated COX activity (curve a) in comparison to the reaction mixtures described above, which corresponds with the fact that here no A2E, which may induce an inhibition, is used. If, however, in addition to A2E, 300 µg/ml of PG is used, this has the result that even in the presence of A2E no inhibition occurs in comparison to the control value (curve b). Rather, the COX activity when PG is used as a negatively charged, "protective" phospholipid in the presence of A2E is even slightly elevated, but there is definitely no longer any inhibitory effect to be seen on the A2E.

### Example 3: Dependence of the A2E-induced inhibition on the phospholipid used for cytochrome c oxidase reconstituted in lipids and prevention of the inhibition by negatively charged phospholipids

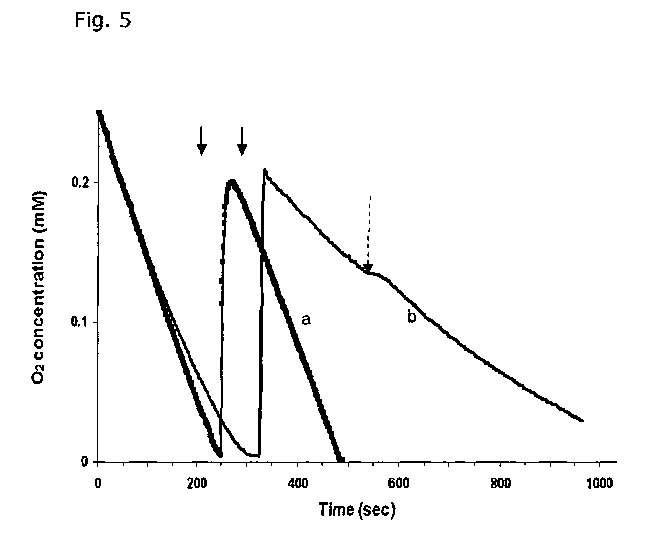

The inhibition of the COX induced by A2E was also investigated using the "natural" environment of the vesicles sharing the cytochrome c oxidase. The reconstitution (see above, under "methods") was successfully performed, the average respiration control index being used as a parameter for this purpose, which was at a value higher than 5.5 without a decoupler. Therefore, successful reconstitution of the COX in the lipid environment of the vesicle may be assumed. While no protection from inhibition using A2E could be established for the reconstitution of COX in vesicles containing phosphatidylcholine/phosphatidylethanolamine (PC/PE), a protective effect was shown when vesicles which contained negatively charged phospholipids were used. Thus, vesicles containing asolectin showed a clear protective effect in regard to reconstituted COX when A2E was used as an inactivator (see Fig. 4). Cytochrome c oxidase was reconstituted in phosphatidylcholine/phosphatidylethanolamine vesicles (squares) or asolectin vesicles (circles). The oxygen consumption of the enzyme (c = 3.8 µg/mL) was measured in a Clarke electrode in the presence and absence of A2E, as described in exemplary embodiment 3. While an inhibition of the COX activity of up to 55% occurred when PC/PE was used as a vesicle environment, when asolectin vesicles were used as an environment for the reconstituted COX, it was shown that a constant value of only approximately 10% inhibition occurred. Furthermore, when asolectin vesicles were used, it was shown that COX did not display sensitivity to A2E in darkness or upon exposure to light. This was in contrast to COX which was reconstituted in PC vesicles. In this case, sensitivity of COX to A2E was shown in the darkness, as well as elevated sensitivity upon exposure to light, which led to progressive inhibition of COX (see Fig. 5). The oxygen consumption of reconstituted cytochrome c oxidase (c = 3.8 µg/mL) was measured in a Clarke electrode in the presence of 30 µM A2E, as described in exemplary embodiment 4. 5 µg of catalase from beef liver with 500 µM of H₂O₂ was added at the positions marked by arrows. At the positions marked by dashed arrows, 300 µg/mL of cardiolipin was added. The experiment was performed in darkness (electrode setup was wrapped in aluminum foil; line a) or under the incidence of light (line b). It should be noted that in the absence of A2E, cytochrome c oxidase is equally active in darkness and under the incidence of light (data not shown).

A protective effect by negatively charged phospholipids was also observed when increasing quantities of cardiolipin (see above, a negatively charged phospholipid) were added to vesicles containing PC/PE (see Table III). Similarly, a pronounced protective effect in regard to inactivation of the COX by A2E was also shown if phosphatidylinositol (PI), a further negatively charged phospholipid, was used in the vesicles. The reconstitution attempts were also performed using COX from rat hearts and COX from *Paracoccus denitrificans.* It was also shown in this case that both enzymes were sensitive to A2E and were protected by acid (i.e., negatively charged at pH 7.0) phospholipids.

If cardiolipin was used, a significant protective effect from A2E-induced apoptosis was also shown. To investigate this effect, MCF-7 cells in culture, the initial cell density being 4 x 10⁴ cells/well, were cultivated for a period from 3 and/or 5 days in the presence and absence of A2E (concentrations respectively 10, 30, 60 µM), with and without CL (the concentration was 500 µg/mL). It was shown that A2E, depending on the dose and time, led to the cells becoming apoptotic. If 60 µM of A2E solution was used, 99% of the cells died off after 5 days. In contrast, if the negatively charged phospholipid according to the present invention (CL in this case) was added, 95% of the cells survived. This shows very clearly that the phospholipids and/or compositions according to the present invention allow protection of the cells from apoptosis. These advantageous properties predestine the phospholipids according to the present invention for use as a medication for treating pathological conditions of the retina, particular for treating AMD and macular degeneration.

### Example 4: Binding of A2E to COX and other proteins; specificity and stoichiometry of the binding

To investigate the hypothesis of whether a molecular interaction occurs between COX and A2E, an acid precipitation of COX was performed after the addition of A2E and radiation with light, and/or after A2E exposure in darkness (see Table IV).

**Table IV**

| **Molecules A2E/molecules protein** | | |
|---|---|---|
| **Protein** | **Darkness** | **Exposure to light** |
| COX | 17.0 +/- 0.5 (n=4) | 12,8 +/-4,7 (n=4) |
| COX + CL | 1.1 +/- 1.3 (n=3) | 2.7 +/- 3.6 (n=4) |
| COX + CL + Cyt.c | 0.7 +/- 0.9 (n=3) | 3.8 +/- 2.3 (n=4) |
| Cyt.c | 0.0 (n=3) | 0.0 (n=3) |
| BSA | 1.6 +/- 0.5 (n=3) | 1.6 +/- 0.5 (n=3) |
| Myoglobin | 0.0 (n=2) | 0.0 (n=2) |

Table IV shows the binding of A2E to various proteins investigated using coprecipitation. [³H]-A2E was added to solubilized COX or other proteins. After 20 minutes of incubation in darkness (setup was wrapped in aluminum foil) or under exposure to light, 10% trichloroacetic acid (final concentration) was added, the precipitate was collected on Millipore filters, and the filters, which were dissolved in solvent, were subsequently examined for radioactivity. CL = cardiolipin (diphosphatidylglycerol, DPC); Cyt. c = cytochrome c; BSA = bovine serum albumin.

The duration of the A2E exposure of the COX in light and/or in darkness was 20 minutes each. As may be seen from Table IV, 17 and 13 molecules of A2E, respectively, were bonded to the COX under the conditions indicated. Binding was, for example, almost completely prevented by DPG (negatively charged phospholipid, see above), since in this case instead of 17 molecules of A2E, an average of only 1 molecule of A2E was bonded by COX. This provides a confirmation on a molecular level for the experimental finding that the negatively charged phospholipids of the present invention allow protection of the COX from A2E-induced inactivation. Of course, this has advantageous effects on the cell metabolism, since COX may operate further in the physiological framework and no apoptosomes are formed. Therefore, it is very probable that the protective effect of the retina originating from the negative phospholipids and compositions of the present invention is primarily achieved by shielding and/or "protection" of the cytochrome c oxidase against the attack of A2E.

## Claims

1. Use of at least one negatively charged phospholipid selected from the group consisting of phosphatidylglycerol, phosphatidylinositol and asolectin for the preparation of a medicament or composition for treating or preventing macular degeneration, wherein said medicament or composition does not comprise cardiolipin.

2. The use according to claim 1, wherein the composition in addition comprises at least one different negatively charged phospholipid selected from the group phosphatidylglycerol, phosphatidylinositol and asolectin.

3. The use according to claim 1 or 2, wherein the composition in addition comprises at least one neutral or positively charged phospholipid.

4. The use according to any one of claims 1 to 3, wherein the composition in addition comprises at least one carotenoid and possibly at least one antioxidant.

5. The use according to claim 4, wherein the at least one carotenoid is selected from lutein and zeaxanthin or is a mixture of lutein and zeaxanthin.

6. The use according to claim 4 or 5, wherein the antioxidant is selected from the group including ubiquinone, vitamin E, and ascorbic acid.

7. The use according to any one of claims 1 to 6, wherein the medicament or composition is to be administered in a physiologically acceptable solution to the eye for the treatment and/or prevention of macular degeneration as eye drops, an eye bath, an injection into the eye, an eye insert, or a semisolid preparation.

8. The use according to any one of claims 1 to 7, wherein the medicament or composition is to be administered in a physiologically acceptable solution to the body for the treatment and/or prevention of macular degeneration via intravascular applications or extravascular applications including oral, intranasal and transdermal administration.

## Patentansprüche

1. Verwendung von mindestens einem negativ geladenen Phospholipid ausgewählt aus der Gruppe bestehend aus Phosphatidylglyzerin, Phosphatidylinosit und Asolectin für die Herstellung eines Medikaments oder einer Zusammensetzung zum Behandeln oder Vorbeugen von Makuladegeneration, wobei das Medikament oder die Zusammensetzung nicht Cardiolipin umfasst.

2. Verwendung nach Anspruch 1, wobei die Zusammensetzung des weiteren mindestens ein anderes negativ geladenes Phospholipid ausgewählt aus der Gruppe Phosphatidylglyzerin, Phosphatidylinosit und Asolectin umfasst.

3. Verwendung nach Anspruch 1 oder 2, wobei die Zusammensetzung des weiteren mindestens ein neutrales oder positiv geladenes Phospholipid umfasst.

4. Verwendung nach einem der Ansprüche 1 bis 3, wobei die Zusammensetzung des weiteren mindestens ein Carotinoid und gegebenenfalls mindestens ein Antioxidans umfasst.

5. Verwendung nach Anspruch 4, wobei das mindestens eine Carotinoid ausgewählt ist aus Lutein und Zeaxanthin oder eine Mischung von Lutein und Zeaxanthin ist.

6. Verwendung nach Anspruch 4 oder 5, wobei das Antioxidans ausgewählt ist aus der Gruppe, die Ubichinon, Vitamin E und Ascorbinsäure einschließt.

7. Verwendung nach einem der Ansprüche 1 bis 6, wobei das Medikament oder die Zusammensetzung dem Auge für die Behandlung und/oder Vorbeugung von Makuladegeneration als Augentropfen, als ein Augenbad, als eine Injektion in das Auge, als Augeneinsatz oder als halbfeste Zubereitung in einer physiologisch verträglichen Lösung zu verabreichen ist.

8. Verwendung nach einem der Ansprüche 1 bis 7, wobei das Medikament oder die Zusammensetzung dem Körper für die Behandlung und/oder Vorbeugung von Makuladegeneration mittels intravaskulärer Anwendungen oder extravaskulärer Anwendungen einschließlich oraler, intranasaler und transdermaler Verabreichung in einer physiologisch verträglichen Lösung zu verabreichen ist.

## Revendications

1. Utilisation d'au moins un phospholipide négativement chargé sélectionné parmi le groupe constitué par un phosphatidylglycérol, un phosphatidylinositol et une asolectine pour la préparation d'un médicament ou d'une composition destiné(e) au traitement ou à la prévention de la dégénérescence maculaire, dans laquelle ledit médicament ou ladite composition ne comprend pas de cardiolipine.

2. Utilisation selon la revendication 1, dans laquelle la composition comprend en outre au moins un phospholipide négativement chargé différent sélectionné parmi le groupe constitué par un phosphatidylglycérol, un phosphatidylinositol et une asolectine.

3. Utilisation selon la revendication 1 ou 2, dans laquelle la composition comprend en outre au moins un phospholipide neutre ou positivement chargé.

4. Utilisation selon l'une quelconque des revendications 1 à 3, dans laquelle la composition comprend en outre au moins un caroténoïde et éventuellement au moins un antioxydant.

5. Utilisation selon la revendication 4, dans laquelle le au moins un caroténoïde est sélectionné parmi le groupe constitué par la lutéine et la zéaxanthine ou est un mélange de lutéine et de zéaxanthine.

6. Utilisation selon la revendication 4 ou 5, dans laquelle l'antioxydant est sélectionné parmi le groupe incluant l'ubiquinone, la vitamine E et l'acide ascorbique.

7. Utilisation selon l'une quelconque des revendications 1 à 6, dans laquelle le médicament ou la composition est destiné(e) à être administrée dans une solution physiologiquement acceptable dans l'oeil, pour le traitement et/ou la prévention de la dégénérescence maculaire, sous forme de gouttes pour les yeux, d'un bain d'oeil, d'une injection dans l'oeil, d'un insert oculaire, ou d'une préparation semi-solide.

8. Utilisation selon l'une quelconque des revendications 1 à 7, dans laquelle le médicament ou la composition est destiné(e) à être administrée dans une solution physiologiquement acceptable dans le corps pour le traitement et/ou la prévention de la dégénérescence maculaire via des applications intravasculaires ou des applications extravasculaires incluant une administration orale, intranasale et transdermique.
